(19) Europäisches Patentamt
European Patent Office
Office européen des brevets

(11) Veröffentlichungsnummer: **0 249 976 B1**

(12) # EUROPÄISCHE PATENTSCHRIFT

(45) Veröffentlichungstag der Patentschrift: **27.02.91**

(51) Int. Cl.[5]: **C07C 53/128**, C07C 61/08, C07C 51/14, B01J 29/28

(21) Anmeldenummer: **87108759.9**

(22) Anmeldetag: **19.06.87**

(54) **Verfahren zur Herstellung von Carbonsäuren.**

(30) Priorität: **19.06.86 DE 3620581**

(43) Veröffentlichungstag der Anmeldung:
**23.12.87 Patentblatt 87/52**

(45) Bekanntmachung des Hinweises auf die Patenterteilung:
**27.02.91 Patentblatt 91/09**

(84) Benannte Vertragsstaaten:
**BE DE FR GB NL**

(56) Entgegenhaltungen:
**EP-A- 0 095 304**
**EP-A- 0 107 385**

**BULLETIN OF THE ACADEMY OF SCIENCES OF THE USSR, Band 28, Nr. 2, Teil 2, Februar 1979, Seiten 343-348, Plenum Publishing Corp., New York, US; A.L. LAPIDUS et al.: "Carbonylation of olefins in the presence of supported palladium catalysts"**

(73) Patentinhaber: **BASF Aktiengesellschaft**
**Carl-Bosch-Strasse 38**
**D-6700 Ludwigshafen(DE)**

(72) Erfinder: **Hoelderich, Wolfgang, Dr.**
**Mannheimer Strasse 18 c**
**D-6710 Frankenthal(DE)**
Erfinder: **Reuvers, Johannes G., Dr.**
**Bahnhofstrasse 50**
**D-6521 Hohen-Suelzen(DE)**
Erfinder: **Kummer, Rudolf, Dr.**
**Kreuzstrasse 6**
**D-6710 Frankenthal(DE)**
Erfinder: **Hupfer, Leopold, Dr**
**Waltershoehe 3**
**D-6701 Friedelsheim(DE)**

EP 0 249 976 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Carbonsäuren aus Olefinen durch katalytische Carbonylierung und Hydratisierung in Gegenwart von Zeolithen des Pentasil-Typs.

Unter den Carbonsäuren besitzen die Neocarbonsäuren große Bedeutung in der chemischen Technik. Neopentansäure (Pivalinsäure) wird z.B. zu tert.-Butylperoxipivalat weiterverarbeitet, das als Polymerisationsstarter für Styrol, Vinylchlorid und Vinylacetat eine breite Anwendung findet.

Niedermolekulare Monoester der Neocarbonsäuren können als Geruchsmittel in Waschmitteln, und die Polyester in Schmierölen Verwendung finden. Die Ester dienen auch als Lösungsmittel. Neocarbonsäuren besitzen auch biologische Aktivität in der Fäulnisverhütung, z.B. Neodecansäure als Wurmbekämpfungsmittel.

Die Herstellung von Carbonsäuren durch Umsetzung von Olefinen mit einer um 1 niedrigeren C-Zahl als die gewünschte Carbonsäure mit Kohlenmonoxid in Gegenwart von Mineralsäuren, Lewis-Säuren, "super acids", Metallcarbonylen oder Metallkomplexen als Katalysatoren unter anschließender hydrolytischer Aufarbeitung des Reaktionsgemisches ist bekannt. Die Verwendung von konz. Schwefelsäure, Phosphorsäure, Flußsäure oder Borfluorid als Katalysatoren ist in DE-PS 942 987, US-PS 2 876 241, US 3 167 585, US 3 282 993 und GB 1 174 209 beschrieben. Als Metallkomplex-Katalysatoren kommen zum Einsatz Pd-Komplexe (DE-PS 2 124 718 und EP-PS 55 875), Rhodium- und Iridium-Komplexe (US-PS 3 637 833), Wolfram- und Molybdän-Nickel-Komplexe (GB-PS 2 099 430) und Cobaltcarbonyle (DE-PS 2 503 996). All diesen bekannten Verfahren ist gemeinsam, daß sie homogenkatalytisch und zweistufig durchgeführt werden.

In US-PS 3 733 362 wird die Carbonylierung von 2 bis 6 C-Olefine in der Gasphase geschützt. Der hierbei verwendete Katalysator ist ein mit z.B. Phosphor- oder Arsen-Liganden komplexiertes Metall der 8. Hauptgruppe, wobei dieser ursprüngliche Homogenkatalysator durch Aufbringen auf $SiO_2$ oder $Al_2O_3$ heterogenisiert wird.

Die wesentlichen Nebenprodukte bei dieser Reaktion sind Oligomere der eingesetzten Olefine, höhere Säuren durch Carbonylierung und Hydratisierung der Oligomere und Carbonsäureester durch Reaktion der entstandenen Säuren mit Olefinen.

Die mit der homogenen Herstellungsweise verbundenen Nachteile ergeben sich aus den Schwierigkeiten bei der Katalysatorabtrennung und Entsorgung.

Bei Einsatz von Metallkomplexkatalysatoren müssen aufgrund ihrer Toxizität besondere Maßnahmen ergriffen werden und die thermische Stabilität und damit Aktivität dieser Katalysatoren ist nur in einem engen Temperaturbereich gegeben.

Die Erfindung betrifft ein Verfahren zur Herstellung von Carbonsäuren der Formel

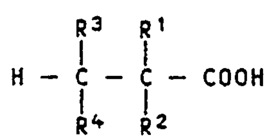

(I)

in der $R^1$ bis $R^4$ Alkyl- und/oder Alkenylreste mit 1 bis 12 Kohlenstoffatomen und/oder Aryl- und/oder Aralkylreste, die ihrerseits substituiert sein können bzw. $R^3$ und $R^4$ Wasserstoff bedeuten bzw. $R^1$ und $R^3$ Teile eines cyclischen Systems sind, durch Umsetzung von Olefinen der Formel

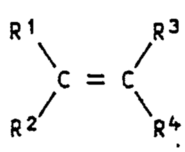

(II)

in der $R^1$ bis $R^4$ die obengenannte Bedeutung haben, mit CO und $H_2O$ in Gegenwart von Zeolithen als Katalysatoren.

Durch das erfindungsgemäße Verfahren können die oben beschriebenen Nachteile vermieden werden, insbesondere dann, wenn die Reaktion in der Gasphase oder unter überkritischen Bedingungen durchgeführt wird.

Als Olefine kann man beispielsweise Isobuten, 2-Methylbuten-1, 2-Methylbuten-2, 2-Methylpenten-1, 2-Methylpenten-2, 2,4-Dimethylbuten-2, 2-Methylhexen-1, 2-Methylocten-1, 2-Methylnonen-1, 2-Methyldecen-1, 2,4,4-Trimethylpenten-1, 2,4,4-Trimethylpenten-2, Cyclopenten, Cyclohexen, 1-Methylcyclopenten, 1-Methylcyclohexen, 1,4-Dimethylcyclopenten, 1,4-Dimethylcyclohexen, 1,3-Dimethylcyclohepten, 1-Methyl-4-phenyl-cyclohexen, 1-Methyl-4-benzyl-cyclohexen, α-Methylstyrol, 1-Phenyl-2-methyl-propen-1 oder 1,1-Diphenylethen verwenden.

Als Katalysatoren eignen sich acide zeolithische Katalysatoren. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekeln besetzt.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

In den Zeolithen können anstelle von Aluminium auch andere Elemente wie B, Ga, Fe, Cr, V, As, Sb oder Be in das Gitter eingebaut werden, oder das Silicium kann durch ein vierwertiges Element wie Ge, Ti, Zr, Hf ersetzt werden.

Für das erfindungsgemäße Verfahren kommen als Katalysatoren Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe in Betracht. In diese Gruppe von Zeolithen gehören auch die sogenannten "ultrastabilen" Zeolithe des Faujasit-Typs, d.h. dealumierte Zeolithe. Verfahren zur Herstellung solcher Zeolithe sind z.B. beschrieben in US-PS 4 512 961.

Besonders vorteilhaft verwendet man Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen. Diese Zeolithe können unterschiedliche chemische Zusammensetzungen aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie um Alumino-, Boro-, Gallium- und Eisengermanatzeolithe oder deren Gemische.

Insbesondere eignen sich die Alumino-, Boro- oder Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyamin-, wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Die erhaltenen Aluminosilikatzeolithe enthalten je nach Wahl ein $SiO_2/Al_2O_3$-Verhältnis von 10 bis 40.000. Hierzu gehören auch die isotaktischen Aluminosilikatzeolithe, wie sie in DE-OS 30 06 471, EP 34727 beschrieben sind. Man kann derartige Aluminosilikatzeolithe auch in etherischem Medium wie Diethylenglykoldimethylester, in alkoholischem Medium wie Methanol bzw. 1,4-Butandiol oder in Wasser synthetisieren.

Die Borosilikatzeolithe kann man z.B. bei 90 bis 200 °C unter autogenem Druck synthetisieren, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Borosilikatzeolithe erhält man auch, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Druck.

Zu den verwendeten siliciumreichen Zeolithen ($SiO_2/Al_2O_3 \geq 10$) gehören z.B. die folgenden Typen: ZSM, Ferrierit und NU-1, als kristalline Zeolithe und Silicalite® als Molekularsieb, ein sog. Silica Polymorph, welches detailiert in US-PS 4 061 724 beschrieben ist.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem

$SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$ sowie Ton. Nach der Verformung werden die Extrudate oder Preßlinge bei 110 °C/16 g getrocknet und bei 500 °C/16 h calciniert.

Man erhält auch vorteilhafte Katalysatoren, wenn der isolierte Aluminobzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs-oder Peptisierungshilfsmittel z.B. Ethylcellulose, Stearinsäure, Kartoffelstärke, Ameisensäure, Oxalsäure, Essigsäure, Salpetersäure, Ammoniak, Amine, Kieselsäureester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden. Wenn bei der Verwendung der zeolithischen Katalysatoren eine, durch Koksabscheidung bedingte, Desaktivierung eintritt, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück. Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden eingesetzt: Alkalimetalle wie Li, K, Cs, Erdalkalimetalle wie Be, Mg, Ca, Metalle der 3., 4. und 5. Hauptgruppe wie Ga, Ge, Sn, Pb, Bi, Übergangsmetalle der 4. bis 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn und Seltene Erdmetalle wie Ce, La, Pr, Nd, Dy, Yb, Lu, Er und U.

Zweckmäßigerweise führt man die Dotierung so durch, daß man z.B. den verformten Zeolithen in einem Steigrohr vorlegt und bei 20 bis 100 °C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2 \times 3 H_2O$ oder $Co(NO_3)_2 \times 6 H_2O$ oder $Ce(NO_3)_3 \times 6 H_2O$ oder $La(NO_3)_3 \times 6 H_2O$ oder $Cs_2CO_3$ in Wasser löst. Mit dieser Lösung wird der verformte oder unverformte Zeolith eine gewisse Zeit, etwa 30 Minuten, getränkt. Die eventuell überstehende Lösung wird am Rotationsverdampfer von Wasser befreit. Danach wird der getränkte Zeolith bei etwa 150 °C getrocknet und bei 550 °C calciniert. Dieser Tränkvorgang kann mehrmals hintereinander vorgenommen werden, um den gewünschten Metallgehalt einzustellen.

Auch ist es möglich, z.B. eine wäßrige $Co(NO_3)_2$-Lösung oder ammoniakalische $Pd(NO_3)_2$-Lösung herzustellen und darin den reinen pulverförmigen Zeolithen bei 40 bis 100 °C unter Rühren etwa 24 h aufzuschlämmen. Nach Abfiltrieren, Trocknen bei etwa 150 °C und Calcinierung bei etwa 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber eine wäßrige $Co(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert.

Bei manchen metalldotierten Zeolithen, z.B. bei Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft so vor, daß man Zeolithe in Pulverform mit 1 n Phosphorsäure 1 Stunde bei 80 °C behandelt. Nach der Behandlung wird mit Wasser gewaschen, bei 110 °C 16 h getrocknet und bei 500 °C 20 h calciniert. Nach einer anderen Arbeitsweise behandelt man Zeolithe vor oder nach ihrer Verformung mit Bindemitteln, z.B. 1 bis 3 h bei Temperaturen von 60 bis 80 °C

mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400 bis 500 °C calciniert.

Eine besondere Ausführungsform für die Säurebehandlung besteht darin, daß man das zeolithische Material vor seiner Verformung bei erhöhter Temperatur mit Flußsäure, im allgemeinen von 0,001 n bis 2 n, vorzugsweise von 0,05 n bis 0,5 n durch Erhitzen unter Rückfluß über einen Zeitraum von 0,5 bis 5, vorzugsweise 1 bis 3 Stunden, behandelt. Nach Isolierung des zeolithischen Materials, z.B. durch Abfiltrieren und Auswaschen, wird dieses zweckmäßigerweise bei Temperaturen von 100 bis 160 °C getrocknet und bei Temperaturen von 450 bis 600 °C calciniert. Eine andere bevorzugte Ausführungsform für die Säurebehandlung besteht darin, daß man das zeolithische Material nach seiner Verformung mit Bindemittel bei Temperaturen von 50 bis 90 °C, vorzugsweise 60 bis 80 °C, über einen Zeitraum von 0,5 bis 5, vorzugsweise 1 bis 3 Stunden mit 3 bis 25 gew.%iger Salzsäure, behandelt. Anschließend kann man das zeolithische Material auswaschen, bei 100 bis 160 °C trocknen und bei 450 bis 600 °C calcinieren. Nach einer anderen Arbeitsweise lassen sich Zeolithe durch Aufbringen von Phosphorverbindungen, wie Trimethylphosphat, Trimethoxyphosphin, primärem, sekundärem oder tertiärem Natriumphosphat modifizieren. Vorteilhaft hat sich die Behandlung mit primärem Natriumphosphat erwiesen. Hierbei werden die Zeolithe in Strang-, Tabletten- oder Wirbelgut-Form z.B. mit wäßriger $NaH_2PO_4$-Lösung getränkt, bei 110 °C getrocknet und bei 500 °C calciniert.

Im allgemeinen können die Katalysatoren wahlweise als Stränge von 2 bis 4 mm Länge, als Tabletten mit 3 bis 5 mm Durchmesser oder als Wirbelgut mit einer Teilchengröße von 0,05 bis 0,5 mm verwendet werden. Das Wirbelgut kann man z.B. durch Zerkleinern und Aussieben von Strängen oder durch Sprühtrocknung herstellen.

Die erfindungsgemäße Umsetzung der Olefine mit CO und $H_2O$ wird bevorzugt in der Gasphase oder im überkritischen Bereich und im allgemeinen bei 50 bis 500 °C, vorzugsweise 200 bis 400 °C und einem Druck von 10 bis 700 bar, vorzugsweise 200 bis 500 bar, ausgeführt. Das molare Verhältnis von Olefinen zu CO und zu $H_2O$ kann zwischen 1:1:1 und 1:10:10, insbesondere zwischen 1:2:2 und 1:6:6 liegen, wobei das Verhältnis CO zu $H_2O$ ebenfalls in diesen Grenzen variieren kann. Die Belastung WHSV kann zwischen 0,1 und 40 $h^{-1}$, bevorzugt 0,5 bis 5 $h^{-1}$ (g Olefin/g Katalysator und h) gewählt werden.

Es ist auch möglich, die Reaktion in der Flüssigphase, z.B. in Suspension-, Riesel- oder Sumpffahrweise, durchzuführen.

Schwerflüchtige oder feste Ausgangsstoffe werden in gelöster Form z.B. in THF-, Toluol- oder Petrolether-Lösung eingesetzt. Im allgemeinen ist eine Verdünnung der Ausgangsstoffe mit derartigen Lösungsmitteln oder Inertgasen wie $N_2$, Ar möglich.

Die Aufarbeitung und Isolierung der Reaktionsprodukte kann in an sich bekannter Weise, z.B. destillativ erfolgen. Nicht umgesetzte Olefine, CO und $H_2O$ kann man abtrennen und erneut für die erfindungsgemäße Umsetzung verwenden.

## Beispiele

Die diskontinuierliche Herstellung der Carbonsäuren wird in einem Rührautoklaven mit 300 ml Füllvolumen durchgeführt. Der Katalysator wird zusammen mit Wasser vorgelegt und danach das Olefin und Kohlenmonoxid zugepumpt. Nach Aufheizen auf die gewünschte Reaktionstemperatur wird Kohlenmonoxid bis zum gewünschten Druck nachgepreßt. Die Reaktionsbedingungen sind in den Beispielen 1 bis 4 angegeben, die Aufarbeitung und weitere Charakterisierung erfolgt wie oben ausgeführt.

Die kontinuierliche Herstellung der Carbonsäuren wird in einem Hochdruck-Rohrreaktor (6 mm Innendurchmesser, 1 m Länge) ausgeführt. Das Olefin wird über eine Hochdruckpumpe in den Reaktor eingespeist. Gleichzeitig erfolgt am Reaktoreingang die getrennte Zugabe von Wasser über eine zweite Hochdruckpumpe bzw. von Kohlenmonoxid (gasförmig). Die Reaktionsbedingungen sind in den einzelnen Tabellen angegeben. Das Reaktionsprodukt wird entspannt, wie angegeben abgetrennt und charakterisiert. Die quantitative Bestimmung der Reaktionsprodukte und der Einsatzstoffe erfolgt gaschromatographisch.

## Katalysator A

Ein Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Aus diesem Material werden durch Verformen mit einem Verformungshilfsmittel 2 mm-Stränge hergestellt, die bei 110 °C/16 h getrocknet und bei 500 °C/24 h calciniert werden.

Katalysator B

Katalysator B erhält man durch Verformung des Borosilikatzeolithen (vgl. Katalysator A) mit Boehmit (Gewichtsverhältnis 60:40) zu 2 mm-Strängen, die bei 110 °C/16 h getrocknet und bei 500 °C/16 h calciniert werden.

Katalysator C

Ein Aluminosilikatzeolith vom Pentasil-Typ wird unter hydrothermalen Bedingungen bei autogenem Druck und 150 °C aus 650 g hochdispersem $SiO_2$, 203 g $Al_2(SO_4)_3 \times 18\ H_2O$ und 10 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Aluminosilikatzeolith enthält 91,6 Gew.% $SiO_2$ und 4,6 Gew.% $Al_2O_3$. Der Katalysator wird mit einem Verformungshilfsmittel zu 2 mm-Strängen verformt, bei 110 °C/16 h getrocknet und bei 500 °C/24 h calciniert.

Katalysator D

Ein Eisensilikatzeolith des Pentasil-Typs wird unter hydrothermalen Bedingungen bei autogenem Druck und 165 °C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 100 °C/24 h getrocknet und bei 500 °C/24 h calciniert. Man erhält einen Eisensilikatzeolithen mit einem $SiO_2/Fe_2O_3$-Verhältnis von 17,7 und einem $Na_2O$-Gehalt von 1,2 Gew.%. Der Zeolith wird mit hochdispersem $SiO_2$ im Gewichtsverhältnis 80:20 zu 2,5 mm-Strängen verstrangt, bei 110 °C/16 h getrocknet und bei 500 °C/24 h calciniert.

Katalysator E

Der Katalysator wird aus Eisensilikatzeolith, wie Katalysator D, durch Verformung mit Verformungshilfsmitteln zu Strängen und anschließende Calcination bei 500 °C/16 h hergestellt. Die Stränge werden mit einer 20 %igen wäßrigen $NH_4Cl$-Lösung bei 80 °C einem Ionenaustausch unterworfen, getrocknet und bei 500 °C/24 h calciniert. Der Vorgang wird mehrmals wiederholt, bis der Na-Gehalt des Katalysators unter 0,03 Gew.% abgefallen ist.

Katalysator F

Der Katalysator wird wie Katalysator C hergestellt, wobei jedoch das 1,6-Hexandiamin durch 1,3-Propandiamin ersetzt wird. Der erhaltene Aluminosilikatzeolith enthält 90,6 Gew.% $SiO_2$ und 3,4 Gew.% $Al_2O_3$.

Das erhaltene Pulver wird mit Boehmit im Gewichtsverhältnis 60:40 verformt, bei 110 °C/16 h getrocknet und bei 500 °C/24 h calciniert.

Katalysator G

Katalysator G wird hergestellt, indem man Katalysator B mit wäßriger $Cr(NO_3)_3$-Lösung imprägniert, bei 130 °C/2 h trocknet und bei 540 °C/2 h calciniert. Der Cr-Gehalt beträgt 2,8 Gew.%.

Katalysator H

wie Katalysator G nur $Cu(NO_3)_2$-Lösung. Der Cu-Gehalt beträgt 3,5 Gew.%.

Katalysator I

wie Katalysator G nur $Cu(NO_3)_2$-Lösung. Der Cu-Gehalt beträgt 1,4 Gew.%.

Katalysator J

Katalysator J wird hergestellt, indem man Katalysator A mit wäßriger $La(NO_3)_3$-Lösung imprägniert, bei 130° C/2 h trocknet und bei 540° C/2 h calciniert. Der La-Gehalt beträgt 1,65 Gew.%.

Katalysator K

wie Katalysator J nur $Ce(NO_3)_2$-Lösung. Der Ce-Gehalt beträgt 1,65 Gew.%.

Katalysator L

wie Katalysator G nur $Co(NO_3)_2$-Lösung. Der Co-Gehalt beträgt 1,7 Gew.%.

Katalysator M

wie Katalysator G nur $Co(NO_3)_2$-Lösung. Der Co-Gehalt beträgt 0,45 Gew.%.

Katalysator N

wie Katalysator G nur eine Lösung aus $Cu(NO_3)_2$ und $Co(NO_3)_2$. Der Cu-Gehalt beträgt 1,8 Gew.% und der Co-Gehalt 1,6 Gew.%.

Katalysator O

wie Katalysator G nur eine Lösung aus $Ca(OH)_2$ und $Co(NO_3)_2$. Der Ca-Gehalt beträgt 0,1 Gew.% und der Co-Gehalt 1,1 Gew.%.

Katalysator P

Katalysator P wird hergestellt, indem man Katalysator C mit wäßriger $Co(NO_3)_2$-Lösung imprägniert, bei 130° C/2 h getrocknet und bei 540° C/2 h calciniert. Der Co-Gehalt beträgt 3 Gew.%.

Katalysator Q

wie Katalysator P nur Gemisch aus $Mg(OH)_2$- und $Co(NO_3)_2$-Lösung. Der Mg-Gehalt beträgt 1 Gew.% und der Co-Gehalt 1,5 Gew.%.

Katalysator R

wie Katalysator G nur $Co(NO_3)_2$-Lösung. Der Co-Gehalt beträgt 3,6 Gew.%.

Beispiele 1 bis 3

Im diskontinuierlichen Versuch werden die Katalysatoren B, F und R getestet. Dazu werden 5 g Katalysator, 54 g Isobuten und 20 g Wasser in den Autoklaven gegeben und der Druck mit CO auf 50 bar eingestellt. Nach Aufheizen auf 300 °C wird der Druck mit weiterem Kohlenmonoxid auf 500 bar eingestellt und durch sukzessive Zugabe von Kohlenmonoxid während der Reaktion konstant gehalten. Nach einer Reaktionszeit von 11 bis 15 h werden bei

| Beispiel | Katalysator | Gew.% Pivalinsäure |
|---|---|---|
| 1 | B | 44 |
| 2 | F | 38 |
| 3 | R | 28 |

im Austrag erhalten.

Beispiel 4

Wie Beispiele 1 bis 3, jedoch werden hierbei 41 g Cyclohexen eingesetzt und die Reaktion bei 300 bar durchgeführt. Nach einer Reaktionszeit von 48 h werden 14 Gew.% Cyclohexancarbonsäure im Austrag gefunden.

Beispiele 5 bis 22

Die Beispiele 5 bis 22 werden kontinuierlich mit Isobuten durchgeführt wie oben beschrieben. Die Reaktionsbedingungen und Ergebnisse sind in Tabelle 1 und 2 zusammengestellt.

Beispiele 23 bis 24

Langzeitversuche zur Reaktion Isobuten mit CO und $H_2O$ am Katalysator N bzw. L über 110 h bzw. 82 h bei 300 °C, 300 bar, WHSV = 2,5 $h^{-1}$ und einem molaren Verhältnis 1:5:5 bzw. 1:3:3 zeigen das in Tabelle 3 bzw. 4 beschriebene Laufzeitverhalten.

Tabelle 3: Katalysator N

| Laufzeit, h | 4 | 19 | 36 | 68 | 88 | 103 |
|---|---|---|---|---|---|---|
| Pivalinsäure im flüssigen Austrag in Gew.% | 58,9 | 59,2 | 66,8 | 64,4 | 64,2 | 66,1 |

Tabelle 4: Katalysator L

| Laufzeit, h | 14 | 27 | 68 | 79 |
|---|---|---|---|---|
| Pivalinsäure im flüssigen Austrag in Gew.% | 53,9 | 53,1 | 47,7 | 48,2 |

Tabelle 1

| Beispiel | 5 | 6 | 7 | 8 | 9 | 10 | 11 | 12 | 13 |
|---|---|---|---|---|---|---|---|---|---|
| Katalysator | B | B | C | E | G | H | I | J | K |
| Temperatur [°C] | 250 | 300 | 300 | 300 | 250 | 250 | 250 | 250 | 250 |
| Druck [bar] | 300 | 300 | 300 | 300 | 300 | 300 | 300 | 300 | 300 |
| WHSV | 2,5 $h^{-1}$ | 2,5 $h^{-1}$ | 2,5 $h^{-1}$ | 2,5 $h^{-1}$ | 2,5 $h^{-1}$ | 2,5 $h^{-1}$ | 2,5 $h^{-1}$ | 2,5 $h^{-1}$ | 2,5 $h^{-1}$ |
| molares Verhältnis 1) | 1:5:5 | 1:5:5 | 1:3:3 | 1:3:3 | 1:3:3 | 1:3:3 | 1:3:3 | 1:3:3 | 1:3:3 |
| Pivalinsäure im flüssigen Austrag in Gew.% 2) | 37,2 | 58,8 | 48,4 | 51,0 | 33,9 | 51,2 | 35,6 | 49,3 | 39,0 |

1) Isobuten : CO : $H_2O$

2) Der Rest setzt sich zusammen aus CO, Isobuten, $H_2O$ und andere organische Verbindungen

Tabelle 2

| Beispiel | 14 | 15 | 16 | 17 | 18 | 19 | 20 | 21 | 22 |
|---|---|---|---|---|---|---|---|---|---|
| Katalysator | M | L | R | N | N | N | O | P | Q |
| Temperatur [°C] | 300 | 300 | 300 | 250 | 300 | 300 | 300 | 300 | 300 |
| Druck [bar] | 300 | 300 | 300 | 300 | 300 | 300 | 300 | 300 | 300 |
| WHSV | 2,5 $h^{-1}$ | 2,5 $h^{-1}$ | 2,5 $h^{-1}$ | 2,5 $h^{-1}$ | 2,5 $h^{-1}$ | 2,5 $h^{-1}$ | 2,5 $h^{-1}$ | 2,5 $h^{-1}$ | 2,5 $h^{-1}$ |
| molares Verhältnis 1) | 1:3:3 | 1:3:3 | 1:3:3 | 1:3:3 | 1:3:3 | 1:5:5 | 1:3:3 | 1:3:3 | 1:3:3 |
| Pivalinsäure im flüssigen Austrag in Gew.% 2) | 68,4 | 53,9 | 40,2 | 35,6 | 51,3 | 72,6 | 55,5 | 46,9 | 54,8 |

1) Isobuten : CO : $H_2O$

2) Der Rest setzt sich zusammen aus CO, Isobuten, $H_2O$ und andere organische Verbindungen

## Ansprüche

1. Verfahren zur Herstellung von Carbonsäuren der Formel

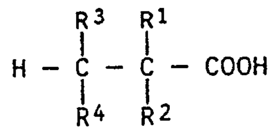

(I)

in der $R^1$ bis $R^4$ Alkyl- bzw. Alkenylreste mit 1 bis 12 Kohlenstoffatomen oder Aryl- bzw. Aralkylreste, die ihrerseits substituiert sein können bzw. $R^3$ und $R^4$ Wasserstoff bedeuten bzw. in der $R^1$ und $R^3$ Teile eines cyclischen Systems sind, durch katalytische Carbonylierung und Hydratisierung, dadurch gekennzeichnet, daß man Olefine der Formel

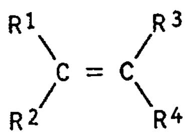

(II)

in der $R^1$ bis $R^4$ die obengenannte Bedeutung haben, mit CO und $H_2O$ in Gegenwart von Zeolithen als Katalysatoren umsetzt.

2. Verfahren nach Anspruch 1, dadurch gekennzeichnet, daß man die Umsetzung bei Temperaturen von 50 bis 500 °C und bei Drucken von 10 bis 700 bar durchführt.

3. Verfahren nach Anspruch 1 und 2 zur Herstellung von Neocarbonsäuren, dadurch gekennzeichnet, daß als Olefine Isoolefine eingesetzt werden.

4. Verfahren nach Anspruch 3, dadurch gekennzeichnet, daß als Isoolefin Isobuten eingesetzt wird.

5. Verfahren nach Anspruch 1 und 2, dadurch gekennzeichnet, daß als Olefin Cyclohexen eingesetzt wird.

6. Verfahren nach Anspruch 1 bis 5, dadurch gekennzeichnet, daß man als Katalysatoren Zeolithe des Pentasil-Typs verwendet.

7. Verfahren nach Anspruch 6, dadurch gekennzeichnet, daß man als Katalysatoren Aluminosilikatzeolithe verwendet.

8. Verfahren nach Anspruch 1 bis 7, dadurch gekennzeichnet, daß man mit Metallen dotierte Zeolithe als Katalysatoren verwendet.

9. Verfahren nach Anspruch 1, dadurch gekennzeichnet, daß man mit Übergangsmetallen dotierte Zeolithe als Katalysatoren verwendet.

10. Verfahren nach Anspruch 1, dadurch gekennzeichnet, daß man mit seltenen Erdmetallen dotierte Zeolithe als Katalysatoren verwendet.

11. Verfahren nach Anspruch 1, dadurch gekennzeichnet, daß man mit Edelmetallen dotierte Zeolithe als Katalysatoren verwendet.

12. Verfahren nach Anspruch 1, dadurch gekennzeichnet, daß man als Katalysatoren Cu- und/oder Co-dotierte Zeolithe einsetzt.

## Claims

1. A process for preparing a carboxylic acid of the formula

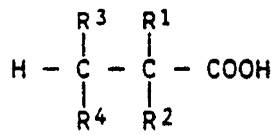

(I)

where $R^1$, $R^2$, $R^3$ and $R^4$ are each alkyl or alkenyl of 1 to 12 carbon atoms, or aryl or aralkyl, which can each in turn be substituted, or $R^3$ and $R^4$ are each hydrogen or where $R^1$ and $R^3$ are each parts of a cyclic system, by catalytic carbonylation and hydration, which comprises reacting an olefin of the formula

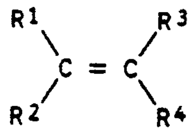

(II)

where $R^1$, $R^2$, $R^3$ and $R^4$ have the abovementioned meanings, with CO and $H_2O$ in the presence of a zeolite of the pentasil type as catalyst at from 50 to 500 °C and at from 10 to 700 bar.

2. A process as claimed in claim 1 for preparing a neocarboxylic acid, wherein the olefin used is an isoolefin.

3. A process as claimed in claim 2, wherein the isoolefin used is isobutene.

4. A process as claimed in claim 1, wherein the olefin used is cyclohexene.

5. A process as claimed in claim 1, wherein the catalyst used is an aluminosilicate zeolite.

6. A process as claimed in claim 1, wherein the catalyst used is a borosilicate zeolite.

7. A process as claimed in claim 1, wherein the catalyst used is an iron silicate zeolite.

8. A process as claimed in any of claims 1 to 7, wherein the catalyst used is a metal-doped zeolite.

9. A process as claimed in claim 1, wherein the catalyst used is a zeolite doped with a transition metal.

10. A process as claimed in claim 1, wherein the catalyst used is a zeolite doped with a rare earth metal.

11. A process as claimed in claim 1, wherein the catalyst used is a zeolite doped with a noble metal.

12. A process as claimed in claim 1, wherein the catalyst used is a Cu- and/or Co-doped zeolite.

## Revendications

1. Procédé de préparation d'acides carboxyliques de formule

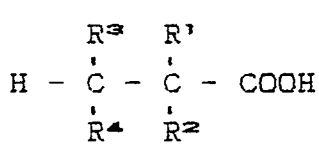

(I)

dans laquelle $R^1$ à $R^4$ représentent des restes alkyle ou alcényle à 1-12 atomes de carbone ou des restes aryle ou aralkyle, qui peuvent être substitués à leur tour, ou bien $R^3$ et $R^4$ sont des atomes d'hydrogène, ou dans laquelle $R^1$ et $R^3$ sont des parties d'un système cyclique, par carbonylation catalytique et hydratation, caractérisé en ce qu'on fait réagir des oléfines de formule

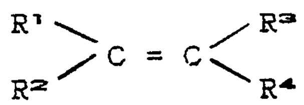

(II)

dans laquelle $R^1$ à $R^4$ ont les significations données cidessus, avec CO et $H_2O$ en présence de zéolithes du type pentasil servant de catalyseurs, à des températures de 50 à 500 °C et sous des pressions de 10 à 700 bars.

**2.** Procédé selon la revendication 1 pour la préparation d'acides néocarboxyliques, caractérisé en ce qu'on fait réagir, en tant qu'oléfines, des iso-oléfines.

**3.** Procédé selon la revendication 2, caractérisé en ce qu'on fait réagir, en tant qu'iso-oléfine, de l'isobutène.

**4.** Procédé selon la revendication 1, caractérisé en ce qu'on fait réagir, en tant qu'oléfine, du cyclohexène.

**5.** Procédé selon la revendication 1, caractérisé en ce qu'on utilise, comme catalyseurs, des zéolithes d'aluminosilicate.

**6.** Procédé selon la revendication 1, caractérisé en ce qu'on utilise, comme catalyseurs, des zéolithes de borosilicate.

**7.** Procédé selon la revendication 1, caractérisé en ce qu'on utilise, comme catalyseurs, des zéolithes de ferrosilicate.

**8.** Procédé selon l'une quelconque des revendications 1 à 7, caractérisé en ce qu'on utilise, comme catalyseurs, des zéolithes dopées avec des métaux.

**9.** Procédé selon la revendication 1, caractérisé en ce qu'on utilise, comme catalyseurs, des zéolithes dopées avec des métaux de transition.

**10.** Procédé selon la revendication 1, caractérisé en ce qu'on utilise, comme catalyseurs, des zéolithes dopées avec des métaux des terres rares.

**11.** Procédé selon la revendication 1, caractérisé en ce qu'on utilise, comme catalyseurs, des zéolithes dopées avec des métaux précieux.

**12.** Procédé selon la revendication 1, caractérisé en ce qu'on utilise, comme catalyseurs, des zéolithes domées avec Cu et/ou Co.